# EUROPÄISCHE PATENTSCHRIFT

(11) **EP 0 581 057 B1**
(45) Veröffentlichungstag und Bekanntmachung des Hinweises auf die Patenterteilung: **07.10.1998**
(21) Anmeldenummer: 93110690.0
(22) Anmeldetag: 05.07.1993
(51) Int. Cl.: A61K 9/70

(54) **Einzeldosierte halbfeste topische Arzneiformen zur Transdermaltherapie**
Pharmaceutical semi-solid topical dosage unit forms for transdermal therapy
Formes de doses unitaires pharmaceutiques topiques semi-solides pour la thérapie percutanée

(30) Priorität: 13.07.1992 DE 4223004
(43) Veröffentlichungstag der Anmeldung: 02.02.1994
(73) Patentinhaber: APL - American Pharmed Labs, Inc., Englewood Cliffs, N.J. 07632 (US)
(72) Erfinder: Liedtke, Rainer K., Dr., c/o Pharmed Dr. Liedtke, D-81379 Gruenwald b. Muenchen (DE)
(74) Vertreter: Tiedtke, Harro, Dipl.-Ing.

(56) Entgegenhaltungen:
- EP-A- 0 262 792
- EP-A- 0 264 552
- EP-A- 0 277 050
- EP-A- 0 429 039

## Beschreibung

Die Erfindung betrifft einzeldosierte halbfeste topische Arzneiformen zur Transdermaltherapie, insbesondere zur Verbesserung der Wirksamkeit und Verträglichkeit systemisch wirkender Arzneistoffe.

Es ist bekannt, daß Arzneistoffe auch auf die Haut appliziert werden. Dies geschieht überwiegend zur Erzielung lokaler Effekte, in selteneren Fällen aber auch zur systemischen Beeinfussung von Körperfunktionen.

Bei letzterem handelt es sich insbesondere um die Anwendung topischer halbfester Arzneiformen insbesondere von Salben, Gelen und Cremes, neuerdings aber vorzugsweise um die Anwendung sogenannter transdermaler therapeutischer Systeme. technisch spezifizierte Pflastersysteme, die bereits zur Behandlung verschiedener Erkankungen erfolgreich eingesetzt werden.

Die transdermale Therapie mit pharmazeutisch üblichen halbfesten topischen Anwendungsformen z.B. im Bereich der Sexualsteroide wie auch die transdermale Anwendung von Nitrogylcerin konnte sich bisher aber therapeutisch nicht durchsetzen, da die Anwendung dieser Formulierungen, insbesondere durch mangelnde Dosiergenauigkeit und Probleme mit der praktischen Handhabung, den Erfordernissen einer systemischen Transdermaltherapie bisher nicht ausreichend gerecht werden konnte. Auch die topische Anwendung von Nitroglycerin mittels eines Sprays konnte sich technisch nicht durchsetzen.

Demgegenüber fanden therapeutische Transdermalsysteme inzwischen aber schon breite Anwendung, insbesondere bei der hormonellen Substitutionstherapie zur Behandlung postmenopausaler Beschwerden und Osteoporose sowie mit Nitroglycerin als symptomatischer Therapie der Angina pectoris bei koronarer Herzkrankheit. Neuere Entwicklungen in diesem Bereich zielen auch auf die Schmerztherapie, z.B. Transdermalsysteme mit dem Analgetikum Fentanyl, sowie auf die Raucherentwöhnung, z.B. mit transdermalen Nikotinpflastern. Verschiedene transdermale Anwendungsbereiche, z.B. transdermales Scopolamin zur Behandlung der Reisekrankheit sowie transdermales Clonidin zur Behandlung der Hypertonie, führten aber auch zu kontroversen Auffassungen zum Nutzen/Risiko-Verhältnis.

Übliche pharmazeutische topische Arzneiformen zeigen wesentlich größere Ungenauigkeit in der Dosierung als Transdermalsysteme. Dies ist, abgesehen von pharmazeutischen Unterschieden in den Formulierungen selbst, überwiegend auf die bisher ungünstige und technisch grobe Applikationstechnik zurückzuführen. So werden hierbei nur sehr grob und unter subjektiver Sicht abgeschätzte Volumina aus den Behältnissen, überwiegend Tuben, teils unter Zuhilfenahme von Spateln mit einer Mess-Skala, appliziert. Die Volumendosierung wird hierbei auch noch von den Patienten selbst vorgenommen.

Diese Vorgehensweise ist, wenn noch überhaupt, nur für unspezifischere Lokaltherapien, z.B. bei Behandlung von lokalen Prellungen mit Analgetika, geeignet. Erhebliche Dosierprobleme ergeben sich auch mit Sprays, da bei diesen nicht nur die genaue Freisetzung der Dosis aus dem Behältnis selbst, sondern auch die entsprechende topische Flächenbelegung problematisch ist. Dies ist teils u.a. bereits durch schwer steuerbare Zerstäubungseffekte in der Luft, teils auch durch Abpralleffekte auf der Haut bedingt.

Zudem benötigt diese Art von Intervention auch spezielle Haftzusätze, die hauttoxikologische Fragen aufwerfen.

Transdermalsysteme sind wesentlich genauer, demgegenüber aber auch produktionmäßig sehr viel aufwendiger und folglich teuer. Weiterhin zeigen Transdermalsysteme auch häufiger Hautirritationen, was durch die für diese Technik erforderliche Hautokklusion der Pflaster erzeugt wird. Zudem sind die Transdermalsysteme, oft aus Kostengründen, auch für längere Anwendungszeiten von mehreren Tagen ausgelegt. Die bekannten Transdermalsysteme sind auch mehr auf vorgegebene konstante Wirkstoff-Liberationsraten fixiert, die sich meist Freisetzungkinetiken nullter Ordnung annähern, somit Kinetiken einer Infusion entsprechen. Dies führt, biologisch erwartungsgemäß, bei einigen Wirkstoffen zu pharmakologischen Gewöhnungsreaktionen. Ein praktisches Beispiel ist hier das transdermale Nitroglycerin, bei dem dies zu reproduzierbarer Tachyphylaxie führt, womit sich bei wiederholter Anwendung die antianginösen Effekte schon kurzfristig, trotz weiter erhöhter Dosis, bis zur Wirkungslosigkeit reduzieren. Für diese Fälle sind entweder undulierende first order Kinetiken oder zeitweise Therapieunterbrechungen, in der die Patienten somit dann unbehandelt bleiben, die derzeitigen Mittel der Wahl zur Wiederherstellung der Wirksamkeit.

Für eine topische Transdermaltherapie systemischer Krankheitszustände sind somit beides, sowohl ausreichend exakte lokale Dosierung als auch Art des Applikationsschemas, zu berücksichtigen.

Für Patient und Arzt ist letztlich auch eine angemessen praktisch durchführbare Art der Anwendung wichtig, die zu differenzierte Vorbereitungen vermeidet, somit eine genügende Fertigkeit der Anwendungsform für die Applikation. Weiterhin sind auch wirtschaftliche Aspekte bezüglich der Kosten einer Arzneitherapie von Belang. Dies gilt insbsondere für chronische Anwendungen.

Die EP-A-0 277 050 (D1) beschreibt ein Verfahren zur Herstellung fester Materialien als Einzeldosierungen, vorgesehen, um Formvorgänge oraler Einzeldosen mit dem nachfolgenden Verpackungsschritt in einem Produktionschritt zu vereinfachen. Verschiedene Rezepturen plastomerer Arzneiformen, die ihren Erweichungspunkt oberhalb Körpertemperatur haben, sind aufgeführt. Solche Festformen können dann aus Blistern herausgedrückt werden.

Die EP-A-0 429 039 beschreibt, in Flüssigform vorliegende, Opiat-Antagonisten in Weichgelatinekapseln, die über Druckausübung rupturieren und deren flüssiger Inhalt dann für eine transdermale Anwendung vorgesehen ist.

Der Erfindung liegt die Aufgabe zugrunde, die Wirksamkeit und Verträglichkeit der topischen Anwendung zur Transdermaltherapie für systemisch wirkende Arzneistoffe zu verbessern.

Diese Aufgabe wird erfindungsmäßig dadurch gelöst, daß die Arzneistoffe als therapeutisch anwendungsfertige topische Einzeldosen einer geeigneten halbfesten Arzneiform, vorzugsweise Creme, Emulsion, Gel, Suspension oder Salbe, oder als therapeutisch anwendungsfertige topische Einzeldosen einer weiteren pharmazeutischen Modifikation der gleichen halbfesten Arzneiform, vorliegen, sich die therapeutisch anwendungsfertigen topischen Einzeldosen in getrennten Behältnissen eines gemeinsamen technischen Formkörpers befinden, der gleichzeitig als Träger für mehrere therapeutisch anwendungsfertige topische Einzeldosen dient, wobei die Wirkstoffe in den therapeutisch anwendungsfertigen topischen Einzeldosen sowohl in verschiedenen Dosierungen, allein, oder auch in Wirkstoffkombinationen, vorliegen können.

Um die Wirksamkeit und Verträglichkeit transdermaler Anwendungen einer systemischen Therapie zu verbessern, können die in dem pharmazeutischen Vehikel enthaltenen Wirkstoffe dabei entweder in einer bestimmten durchschnittlich uniformen Kristallgröße oder auch in Gemischen mit verschiedenen Kristallgrößen vorliegen.

Um die Wirksamkeit und Verträglichkeit transdermaler Anwendungen einer systemischen Therapie mit Steroiden zu verbes sern, sind, in einer weiteren Ausbildung der Erfindung, in der einzeldosierten topischen Arzneiform als Wirkstoffe Östrogene und Gestagene oder Glucocorticoide als Einzelstoffe oder in Kombinationen enthalten.

Um die Wirksamkeit und Verträglichkeit transdermaler Anwendungen einer systemischen Therapie mit Peptiden zu verbessern, sind, in einer weiteren Ausbildung der Erfindung, in der einzeldosierten topischen Arzneiform pharmakologisch wirksame Peptide und Proteine, insbesondere Insulin, Oxytocin, Enkephaline, als Einzelstoffe oder in Kombinationen enthalten.

Um die Wirksamkeit und Verträglichkeit einer transdermalen systemischen Schmerz- oder Rheumatherapie zu verbessern, sind, in einer weiteren Ausbildung der Erfindung, in der einzeldosierten topischen Arzneiform Analgetika und Lokalanästehika wie Buprenorphin, Fentanyl, Penzocain, Morphin und Morphinderivate, Lidocain, Prilocain, Mepivacain oder nichtsteroidale Antirheumatika/Antiphlogistika wie Indomethacin, Diclofenac, Etofenamat als Einzelstoffe oder in Kombinationen enthalten.

Um die Wirksamkeit und Verträglichkeit einer transdermal systemischen Raucherentwöhnung zu verbessern, ist, in einer weiteren Ausbildung der Erfindung, in der einzeldosierten topischen Arzneiform Nikotin enthalten.

Um die praktische Handhabung der einzeldosierten topischen Arzneiform weiter zu verbessern, sind, in einer weiteren Ausbildung der Erfindung, auf dem als gemeinsamen Träger dienenden Formkörper entsprechende Positionen der topischen Einzeldosierungen in einem Therapieschema mit numerischen, farblichen oder symbolischen Angaben und Hinweisen gekennzeichnet.

Um die praktische Anwendung der einzeldosierten topischen Arzneiform weiter zu verbessern, sind in einer weiteren Ausbildung der Erfindung, therapeutische Einzeldosen für verschiedene Therapiephasen aus dem als gemeinsamen Träger dienenden technischen Formkörper separat abtrennbar.

Die mit der Erfindung erzielten Vorteile bestehen insbesondere darin, daß die bekannten therapeutischen Vorteile transdermaler Anwendung übernommen werden können, z.B verringerter systemischer hepatischer first pass Effekt, gleichzeitig aber die bisherige Ungenauigkeit üblicher topischer Arzneiformen, signifikant reduziert wird.

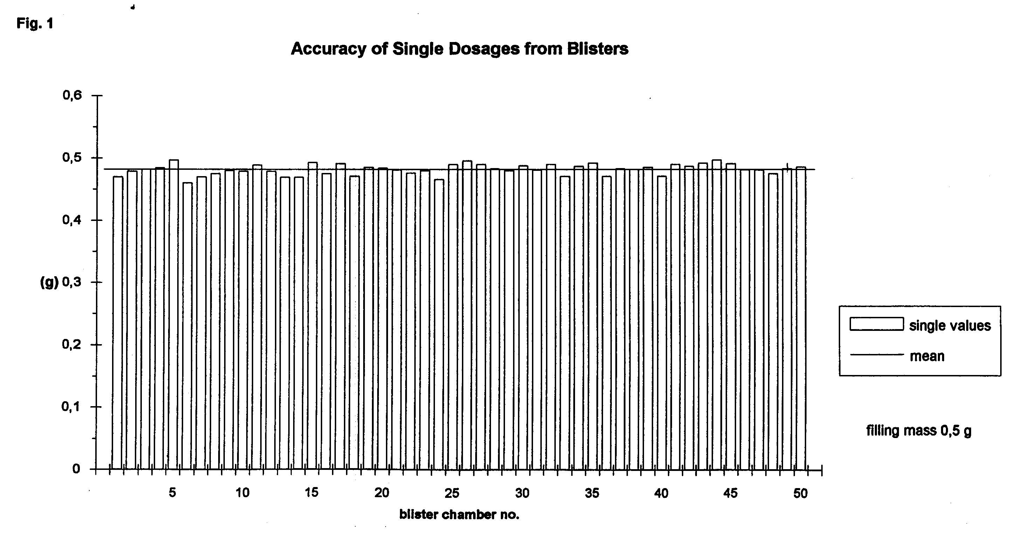

So ließ sich in einer Laboruntersuchung bei 50 manuellen Einzelentnahmen aus vorgefüllten Blisterkammern für eine Estrogen-haltige Formulierung bei einer Einzelfüllmenge von 0,5 Gramm eine Entnahmegenauigkeit der topischen Formulierung mit einer relativen Standardabweichung von 1,9 % reproduzieren (Fig. 1). Dies liegt signifikant unter der für Arzneimittel seitens GMP Procedure geforderten Grenze von 5%. Demgegenüber fand sich bei der Messung der Reproduzierbarkeit der Entnahme für eine konventionelle topische Gelformulierung mit Estrogen aus einer üblichen Tube eine relative Standardabweichung von über 15%.

Die einzeldosierte topische Arzneiform ermöglicht zudem ein individualisiertes und variables Vorgehen. Sie erlaubt vorgefertigte exakte Dosierungsänderungen wie auch vorgefertige und exakt dosierte Kombinationen. Hiermit steht sie in ihrer technischen Anwendung auch in einer stärkeren Analogie zur Vorgehsweise bei oralen Anwendungsformen.

Die definierten Gesamtverhältnisse der einzeldosierten topischen Arzneiform, die sich aus den spezifizierten physikalisch-chemischen Kenndaten der Arzneiform, z.B. physikalischem Spreitverhalten auf der Haut und Permeation in die Haut, der exakten topischen Einzeldosis sowie dem vorgebenen Verordnungschema ergeben, ermöglichen gegenüber üblichen topischen Anwendungen eine wesenlich exaktere Effektsteuerung, somit auch deutlich höhere Therapiesicherheit als bisher.

Darüber hinaus ist die einzeldosierte topische Arzneiform auch deutlich hygienischer und besser schützbar gegen mikrobielle Einflüsse, sowohl bezüglich der Applikation selbst als auch bezüglich ihrer Lagerungsbedingungen.

Die einzeldosierte topische Arzneiform ist im Vergleich zu Transdermalpflastern sehr kostengünstig, was inbesondere auch aus ihrem geringeren Konfektionierungsaufwand in der Massenproduktion resultiert. Sie ist zudem mit üblichen pharmazeutischen Mitteln wie auch unter allen üblichen produktiontechnischen Kautelen, z.B. aseptische oder sterile Erstellung, als auch mit geeigneter Stabilität herstellbar.

Gegenüber technischen Transdermalsystemen ist die Hautverträglichkeit der einzeldosierten topischen Arzneiform grundsätzlich besser.

Differenzierungen der einzeldosierten topischen Arzneiform sind durch Art und Ausgestaltung eines auf dem technischen Behältnis fixierbaren Therapieschemas programmierbar, wobei beispielsweise gleichzeitig verschiedene Dosierungen als auch Wirkstoffkombinationen möglich sind, z.B. von Östrogenen und Östrogen-Gestagen-Kombinationen.

Mit Variationen in den Verteilungen der Kristallgrößen des enthaltenen Wirkstoffes in den topischen pharmazeutschen Formurlierungen lassen sich, durch hieraus resultierendes unterschiedliches Permeations- und Depotverhalten, auch unterschiedliche pharmakokinetische Profile erzeugen, z.B. Retardeffekte.

Das Ausführungsbeispiel einer auf der Erfindung beruhenden Therapiepackung, hier für eine einzeldosierte topische Anwendung zur Hormonsubstitution bei postmenopausalen Beschwerden, sei aufgeführt. Dieses Beispiel soll die Erfindung nur erläutern ohne sie hierauf zu beschränken. Es dient primär dazu eine therapeutische Möglichkeit darzustellen als auch den Grad der Spezifität der mit einzeldosierten topischen Arzneiformen in einer transdermal systemischen Therapie erreichbar wird.

Beispiel: Die topische Therapiepackung besteht aus einem als Packungsträger dienenden Formkörper aus für pharmazeutische Zwecke üblichem Blistermaterial. Ein einzelner Formkörper enthält hierbei 14 als Einzelbehältnisse dienende halbrund ausgeformte Kammern für topische Einzeldosen. Diese können Füllmassen im Bereich von 0,1 bis 1 Gramm aufnehmen. Die Therapiepackung für einen Monat beinhaltet dabei 2 dieser Blisterträger mit jeweils 14 Kammern für eine 28tägige Therapie bei einer täglichen Einzeldosis. In den Kammern befinden sich die anwendungsfertigen therapeutischen Einzeldosen der Formulierung mit 0,5 Gramm. Die eizelnen Kammern sind durchlaufend von 1 bis 28 numeriert. Der Blisterträger 1 mit den Kammern 1-14 enthält dabei in jeweils 0,5 Gramm topischem Vehikel jeweils 3 mg des Estrogens 17β-Estradiol, der Blister 2 mit den Kammern 15-28 enthält dabei in jeweils 0,5 Gramm topischem Vehikel eine Kombination von 3 mg des Estrogens 17β-Estradiol und 1 mg des Gestagens Norethisteron Acetat.

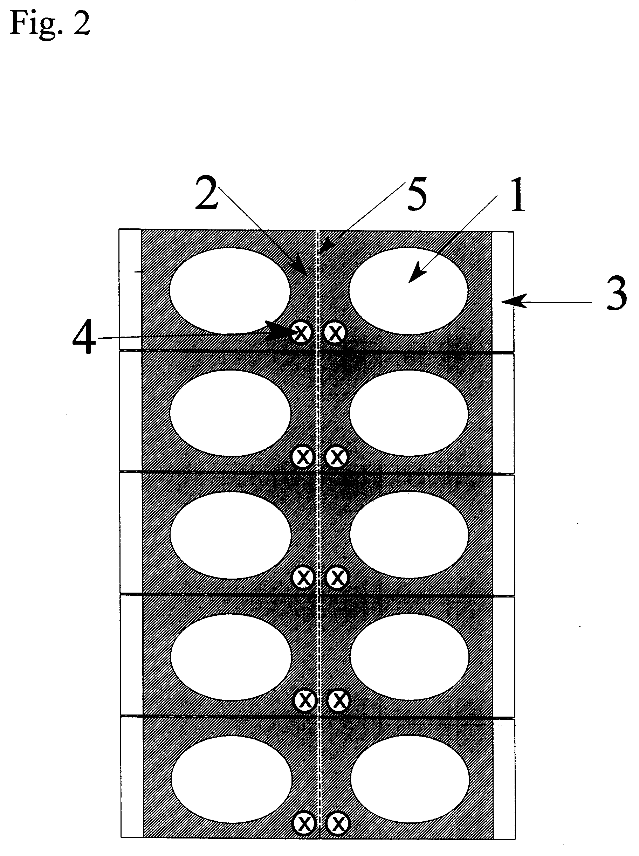

Der Ausschnitt einer Therapiepackung mit topischen Einzeldosierungen ist zur weiteren Erläuterung in Fig. 2 dargestellt, wobei diese Darstellung die Packung nur erläutert ohne sie jedoch in ihrer weiteren Ausgestaltung zu beschränken.

Die einzelnen Blisterkammern der Therapiepackung (1), die die topischen Einzeldosen enthalten, sind an ihrer Oberseite mit einer Peel off Folie (2) aus Aluminium luftdicht versiegelt. Die Peel off Folien sind dabei so angebracht, daß sie an ihrer Aussenseite Laschen (3) besitzen mit der jede Kammer für eine Einzeldosis auch einzeln geöffnet werden kann. Die Abdeckfolie kann dabei an verschiedenen Stellen mit Ziffern (4) für jeden Tag oder auch zusätzlich mit Farbcodes für die Art der Therapiephase versehen sein. Die Therapiepackung ist mit Perforationen (5) versehen, die es erlauben Einzeldosen in ungeöffneter Form von der Gesamtpackung abzutrennen.

Die in den Einzelkammern enthaltenen therapeutischen Einzeldosen werden, wie auch im Schema einer Tabletteneinnahme, täglich entnommen und direkt auf die Haut aufgetragen. Hierbei ergibt sich, auf Grund des spezifizierten Spreitverhaltens des Vehikels auch eine statistisch durchschnittliche Hautflächenbelegung.

Die Volumenbelegung der Einzeldosis-Behältnisse sowie der statistisch durchschnittliche Entnahmerückstand wird bei der Produktion überprüft und produktionstechnisch entsprechend berücksichtigt.

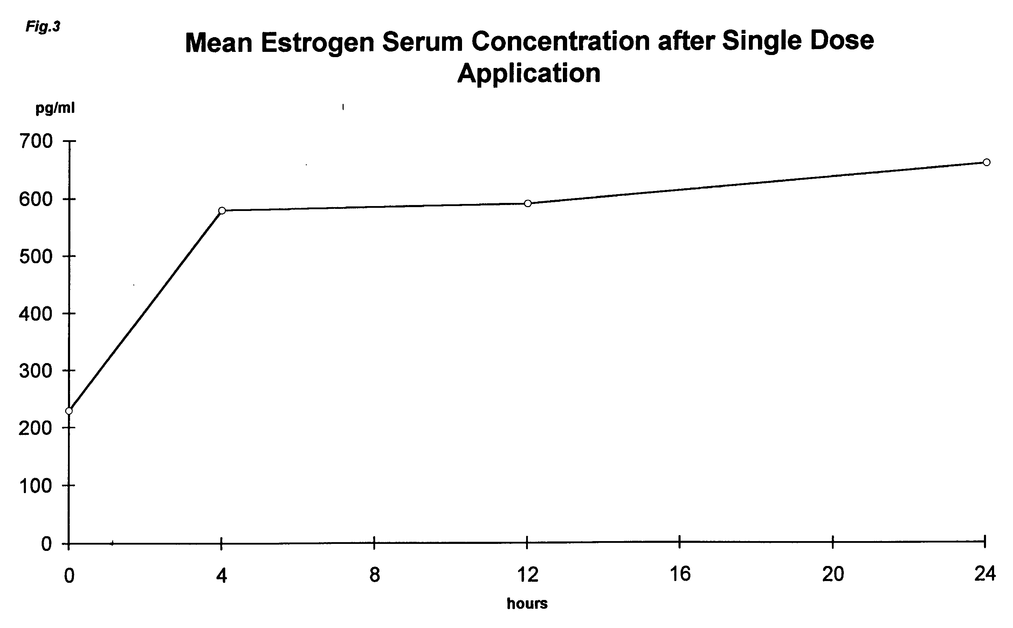

Mit einer einzeldosierten topischen Arzneiform wurde eine klinische Pilotprüfung mit einem Estrogen, Estriol, an 4 Probanden durchgeführt. Das Ergebnis ist in Fig. 3 dargestellt. Es stellt den mittleren Verlauf der Serumkonzentration an radioimmunologisch gemessenem Gesamtestriol über einen Zeitraum von 24 Stunden nach der topischen Applikation dar. Hieraus ergibt sich, daß mit einer einzelnen topischen Dosierung auf die Haut ein über 24 Stunden anhaltendend erhöhter Spiegel an Steroidhormon erreichbar ist. Zur Verbesserung der Wirksamkeit und Verträglichkeit üblicher topischer Applikationen für transdermal systemisch wirkende Arzneistoffe werden aus geeigneten halbfesten Arzneiformen einzeldosierte topische Arzneiformen gebildet, die therapeutisch exakt anwendungsfertig sind. Die topischen Einzeldosen sind bezüglich ihrer Dosis, ihres topischen Spreitverhaltens und ihrer Permeationseigenschaften pharmazeutisch spezifiziert. Mehrere der topisch anwendungsfertigen Einzeldosen sind dabe in einem gemeinsamen technischen Trägerbehältnis untergebracht. Mittels unterschiedlicher Einzelddosierungen oder auch Wirkstoffkombinationen sind differenzierte Therapien gestaltbar.

Als pharmakologische Wirkstoffe werden insbesondere Steroide, Peptide, verschiedene Analgetika, Lokalanästhetika und nichsteroidale Antirrheumatika eingesetzt.

Die einzeldosierte topische Arzneiform ist eine sichere, anwendungsfreundliche und kostengünstige Applikationsform, die eine genauere topische Therapie für systemische Anwendungen ermöglicht als dies bisher mit konventionellen topischen Anwendungsformen erreichbar ist.

## Patentansprüche

1. Einzeldosierte halbfeste topische Arzneiformen zur Transdermaltherapie, insbesondere zur Verbesserung der Wirksamkeit und Verträglichkeit systemisch wirkender Arzneistoffe, **dadurch gekennzeichnet**,
daß die Arzneistoffe als therapeutisch anwendungsfertige topische Einzeldosen einer geeigneten halbfesten Arzneiform, als Creme, Emulsion, Gel, Suspension oder Salbe vorliegen, sich die therapeutisch anwendungsfertigen Einzeldosen in getrennten Behältnissen eines gemeinsamen technischen Formkörpers einer Blisterpackung befinden, der gleichzeitig als Träger für mehrere therapeutisch anwendungsfertige topische Einzeldosen dient und wobei die Wirkstoffe in den therapeutisch anwendungsfertigen topischen Einzeldosen sowohl in verschiedenen Dosierungen, allein oder auch in Wirkstoffkombinationen, vorliegen können.

2. Einzeldosierte halbfeste topische Arzneiformen zur Transdermaltherapie, nach Anspruch 1, dadurch gekennzeichnet, daß die in dem pharmazeutischen Vehikel enthaltenen Wirkstoffe entweder in einer bestimmten uniformen durchschnittlichen Kristallgröße oder auch in Gemischen mit verschiedenen Kristallgröße vorliegen können.

3. Einzeldosierte halbfeste topische Arzneiformen zur Transdermaltherapie, nach Anspruch 1 und 2, dadurch gekennzeichet, daß als Wirkstoffe Östrogene und Gestagene oder Glucocorticoide als Einzelstoffe oder in Kombinationen enthalten sind.

4. Einzeldosierte halbfeste topische Arzneiformen zur Transdermaltherapie, nach Anspruch 1 und 2, dadurch gekennzeichnet, daß als Wirkstoffe pharmakologisch wirksame Peptide oder Proteine, insbesondere Insulin, Oxytocin, Enkephaline, als Einzelstoffe oder als Kombinationen eingesetzt sind.

5. Einzeldosierte halbfeste topische Arzneiformen zur Transdermaltherapie, nach Anspruch 1 und 2, dadurch gekennzeichnet, daß als chemische Stoffe Analgetika und Lokalanästhetika, insbesondere Buprenorphin, Fentanyl, Penzocain, Morphin oder Morphinderivate, Lidocain, Prilocain, Mepivacain, oder nichsteroidale Antirheumatika /Antiphlogistika, insbesondere Indomethacin, Diclofenac, Etofenamat als Einzelstoffe oder in Kombinationen eingesetzt sind.

6. Einzeldosierte halbfeste topische Arzneiformen zur Transdermaltherapie, nach Anspruch 1 und 2, dadurch gekennzeichnet, daß als Wirkstoff Nikotin enthalten ist.

7. Einzeldosierte halbfeste topische Arzneiformen zur Transdermaltherapie, nach vorhergehenden Ansprüchen, dadurch gekennzeichnet, daß auf dem als gemeinsamen Träger dienenden Formkörper entsprechende Positionen der topischen Einzeldosierungen in einem Therapieschema mit numerischen, farblichen oder symbolischen Angaben und Hinweisen gekennzeichnet sind.

8. Einzeldosierte halbfeste topische Arzneiformen zur Trandermaltherapie, nach vorhergehenden Ansprüchen, dadurch gekennzeichnet, daß therapeutische Einzeldosen für verschiedene Therapiephasen aus dem als gemeinsamen Träger dienenden technischen Formkörper separat abtrennbar sind.

## Claims

1. Pharmaceutical semi-solid topical dosage unit forms for transdermal therapy, in particular for improving the effectiveness and compatibility of systemically effective pharmaceuticals, **characterised in that** the pharmaceuticals are available as therapeutically ready-for-use topical dosage units of a suitable semi-solid form of medication as creme, emulsion, gel, suspension or paste, that the therapeutically ready-for-use dosage units are held in separate containers of a common technical shape of a blister package which at the same time serves as carrier for a plurality of therapeutically ready-for-use topical dosage units, and the active ingredients can be present in the therapeutically ready-for-use topical dosage units both in different dosages, singly or in a combination of active ingredients.

2. Pharmaceutical semi-solid topical dosage unit forms for transdermal therapy according to Claim 1, **characterised in that** active ingredients contained in the pharmaceutical vehicle can be present either in a specified uniform average crystal size or also in a mixture of different crystal sizes.

3. Pharmaceutical semi-solid topical dosage unit forms for transdermal therapy according to Claim 1 and 2, **characterised in that** estrogen and gestagen or glucocorticoide as active ingredients are contained as individual substances or in a combination.

4. Pharmaceutical semi-solid topical dosage unit forms for transdermal therapy according to Claim 1 and 2, **characterised in that** pharmacologically effective peptides or proteins, in particular insulin, oxytocin, enkephaline, are used as active ingredients either as individual substances or in a combination.

5. Pharmaceutical semi-solid topical dosage unit forms for transdermal therapy according to Claim 1 and 2, **characterised in that** the following chemical substances are applied as individual substances or in combinations: analgetics and local anaesthetics, in particular Buprenorphin, Fentanyl, Penzocain, morphine or morphine derivatives, Lidocain, Prilocain, Mepivacain, or non-steroidal anti-rheumatics/anti-phlogistics, in particular Indomethacin, Diclofenac, Etofenamat.

6. Pharmaceutical semi-solid topical dosage unit forms for transdermal therapy according to Claim 1 and 2, **characterised in that** it contains nicotine as an active ingredient.

7. Pharmaceutical semi-solid topical dosage unit forms for transdermal therapy according to the above claims, **characterised in that** on the shape which serves as common carrier respective positions of the topical individual dosages are characterised in a therapy diagram with numeric, colour or symbolic particulars and references.

8. Pharmaceutical semi-solid topical dosage unit forms for transdermal therapy according to the above claims, **characterised in that** therapeutic individual dosages for different therapy phases are separable from the technical shape which serves as common carrier.

## Revendications

1. Formes pharmaceutiques topiques semi-solides à doses unitaires pour la thérapie percutanée, notamment pour améliorer l'activité et la compatibilité de substances actives à action systémique, caractérisées en ce que les substances actives sont présentes comme doses unitaires topiques, prêtes à l'usage thérapeutique, d'une forme pharmaceutique semi-solide appropriée telle que crème, émulsion, gel, suspension ou pommade, les doses unitaires prêtes à l'usage thérapeutique se trouvent dans des compartiments séparés d'un corps commun techniquement formé d'un emballage blister, qui sert en même temps de support pour plusieurs doses unitaires topiques prêtes à l'usage thérapeutique et où les substances actives contenues dans les doses unitaires topiques prêtes à l'usage thérapeutique peuvent être présentes aussi bien à diverses doses, seules, que dans des associations de substances actives.

2. Formes pharmaceutiques topiques semi-solides à doses unitaires pour la thérapie percutanée suivant la revendication 1, caractérisées en ce que les substances actives contenues dans le véhicule pharmaceutique peuvent être présentes avec une dimension moyenne uniforme déterminée des cristaux ou aussi en mélanges avec une dimension variée des cristaux.

3. Formes pharmaceutiques topiques semi-solides à doses unitaires pour la thérapie percutanée suivant les revendications 1 et 2, caractérisées en ce qu'elles contiennent comme substances actives des oestrogènes ou des progestatifs ou des glucocorticoïdes comme substances individuelles ou en associations.

4. Formes pharmaceutiques topiques semi-solides à doses unitaires pour la thérapie percutanée suivant les revendications 1 et 2, caractérisées en ce qu'on utilise comme substances actives des peptides ou protéines pharmacologiquement actives, notamment de l'insuline, de l'oxytocine, des encéphalines, individuellement ou en associations.

5. Formes pharmaceutiques topiques semi-solides à doses unitaires pour la thérapie percutanée suivant les revendications 1 et 2, caractérisées en ce qu'on utilise comme substances chimiques des analgésiques et des anesthésiques locaux, en particulier la buprénorphine, le fentanyl, la penzocaïne, la morphine ou des dérivés de morphine, la lidocaïne, la prilocaïne, la mépivacaïne ou des antirhumatismaux/antiphlogistiques non stéroïdiens, en particulier l'indométhacine, le diclofénac, l'étofénamat, comme substances individuelles ou en associations.

6. Formes pharmaceutiques topiques semi-solides à doses unitaires pour la thérapie percutanée suivant les revendications 1 et 2, caractérisées en ce qu'elles contiennent la nicotine comme substance active.

7. Formes pharmaceutiques topiques semi-solides à doses unitaires pour la thérapie percutanée suivant les revendications précédentes, caractérisées en ce que des positions correspondantes des doses individuelles topiques dans un schéma thérapique sont signalées par des nombres, des couleurs ou des symboles indicatifs sur le corps façonné servant de support commun.

8. Formes pharmaceutiques topiques semi-solides à doses unitaires pour la thérapie percutanée suivant les revendications précédentes, caractérisées en ce que des doses thérapeutiques individuelles pour les diverses phases de la thérapie peuvent être détachées séparément du corps façonné techniquement servant de support commun.
